(19) **Europäisches Patentamt**

**European Patent Office**

**Office européen des brevets**

(11) Veröffentlichungsnummer : **0 315 690 B1**

(12) **EUROPÄISCHE PATENTSCHRIFT**

(45) Veröffentlichungstag der Patentschrift :
08.01.92 Patentblatt 92/02

(21) Anmeldenummer : **87906032.5**

(22) Anmeldetag : **26.05.87**

(86) Internationale Anmeldenummer :
**PCT/SU87/00061**

(87) Internationale Veröffentlichungsnummer :
**WO 88/09349 01.12.88 Gazette 88/26**

(51) Int. Cl.⁵ : **C08F 220/56, C08F 261/02, C12N 1/00**

(54) **VERFAHREN ZUR HERSTELLUNG EINES FESTEN MEDIUMS ZUR KULTIVIERUNG VON MIKROORGANISMEN.**

(43) Veröffentlichungstag der Anmeldung :
**17.05.89 Patentblatt 89/20**

(45) Bekanntmachung des Hinweises auf die
Patenterteilung :
**08.01.92 Patentblatt 92/02**

(84) Benannte Vertragsstaaten :
**AT BE CH DE FR GB IT LI LU NL SE**

(56) Entgegenhaltungen :
**GB-A- 2 162 198**
**SU-A- 514 893**
**SU-A- 977 466**
**Keine Entgegenhaltungen.**

(73) Patentinhaber : **KIEVSKY MEDITSINSKY INSTITUT IMENI AKADEMIKA A.A.BOGOMOLTSA**
**Bulvar T.G.Shevchenko, 13**
**Kiev, 252004 (SU)·**

(72) Erfinder : **GASHINSKY, Vladimir Vladislavovich**
**ul. Repina, 7/13-11**
**Kiev, 252004 (SU)**
Erfinder : **KRAINJUKOVA, Tatyana Ivanovna**
**ul. Zakrevskogo, 11-120**
**Kiev, 252222 (SU)**
Erfinder : **KOKOSHA, Natalya Vladimirovna**
**ul. Polyarnaya, 3-168**
**Kiev, 252200 (SU)**
Erfinder : **PIVOVAREVICH, Lidia Petrovna**
**pr. Gagarina, 145**
**Kharkov, 310035 (SU)**

(74) Vertreter : **von Füner, Alexander, Dr. et al**
**Patentanwälte v. Füner, Ebbinghaus, Finck**
**Mariahilfplatz 2 & 3**
**W-8000 München 90 (DE)**

**Beschreibung**

Technisches Gebiet

Die vorliegende Erfindung bezieht sich auf das Gebiet der Medizin und Biologie und betrifft insbesondere ein Verfahren zur Herstellung eines Platten-Nährmediums auf Grundlage eines synthetischen Gels zur Kultivierung verschiedener Arten der Mikroorganismen.

Zugrundeliegender Stand der Technik

Es ist ein Verfahren zur Herstellung eines Platten-Nährmediums für medizinische und mikrobiologische Zwecke durch die radikalische Kopolymerisation von Akrylamid und N,N'-Methylen-bis-akrylamid in einer physiologischen Lösung bei deren Massenverhältnis von 0,5-40,0 bzw. 2,5-12,0 bis zur Bildung eines Polyakrylamidgels bekannt. Das gewonnene Gel, das die maximale Wassermenge enthält, wird mit der physiologischen Lösung durchgewaschen und mit Nährsubstraten imprägniert (SU-A-977466).

Das erhaltene Platten-Närmedium wird zu Untersuchung biologischer Eigenschaften der Mikroorganismen verwendet. Jedoch wird das genannte Platten-Nährmedium durch eine hohe Verletzbarkeit der oberflächlichen Schicht bei der Sterilisation, der Impfung der Mikroorganismen darauf sowie bei der Abnahme der Kolonie mit der bakteriologischen Öse gekennzeichnet.

Die Abnahme der Mikroorganismen bei wiederholten Überimpfungen wird durch das Abspülen mit der physiologischen Lösung verwirklicht, was bei den meisten mikrobiologischen Arbeiten nicht angebracht ist.

Es ist auch eine Verfahren zur Herstellung eines Platten-Nährmediums zur Kultivierung der Mikroorganismen bekannt, das darin besteht, dass die radikalische Kopolymerisation von Akrylamid mit N,N'-Methylen-bis-akrylamid in der physiologischen Lösung bei deren Massenverhältnis von 15-20 bzw. 0,019-0,132 bis zur Bildung eines Polyakrylamidgels durchgeführt wird. Das gewonnene Gel wird mit der physiologischen Lösung abgewaschen und danach der Quellung in der physiologischen Lösung innerhalb von 16 bis 20 Stunden bei einer Temperatur von 50 bis 60°C unterzogen. Hiernach wird das Polyakrylamidgel mit Nährsubstraten imprägniert (DE, A, 3430316).

Das erhaltene Platten-Nährmedium wird zur Kultivierung der Mikroorganismen verwendet. Es ist elastisch, durchsichtig und besitzt gute Adhäsionseigenschaften. Die Mikroorganismenkulturen werden an dessen Oberfläche angeordnet, ohne darin hineinzuwachsen, und lassen sich vollständig mit der bakteriologischen Öse abnehmen. Jedoch wird an der Oberfläche dieses Platten-Nährmediums während dessen Aufbewahrung in den Petrischalen und Inkubation Ausschwitzung der Flüssigkeit nachgewiesen, die bei einer längeren Abtrocknung im Thermostat nicht verschwindet. Ausserdem ergeben einige Mikroorganismenarten, insbesondere bewegliche Formen, auf dem genannten Nährmedium kein typisches Kolonienwachstum bei kennzeichnenden morphologischen, biochemischen, antigenen Eigenschaften. Dies schränkt die Verwendung des oben beschriebenen Nährmediums, beispielsweise zur Diagnostik der Infektionserkrankungen, ein.

Der Erfindung liegt die Aufgabe zugrunde, ein Verfahren zur Herstellung solch eines Platten-Nährmediums durch Einführung eines Reagens für die zusätzliche Strukturierung dem Polyakrylamidgels zu entwickeln, das die Möglichkeit des Wachstums eines breiten Spektrums der Mikroorganismen mit typischen biologischen Eigenschaften sichern würde.

Die Aufgabe wird dadurch gelöst, dass ein Verfahren zur Herstellung des Platten-Nährmediums zur Kultivierung der Mikroorganismen vorgeschlagen wird, das die radikalische Kopolymerisation von Akrylamid mit N,N'-Methylen-bis-akrylamid bei deren Massenverhältnis von (15-20):(0,019-0,132) in einer physiologischen Lösung bis zur Bildung eines Gels, das Abspülen und dessen Quellung in der physiologischen Lösung, die Imprägnierung mit Nährsubstraten vorsieht, und in welchem erfindungsgemäss die radikalische Kopolymeristion in Gegenwart des Polyvinylalkohols bei einem Gewichtsverhältnis von Akrylamid und Polyvinylalkohol von 15-20:1,0-3,0 durchgeführt wird.

Polyvinylalkohol kann als wäßrige Lösung verwendet werden.

Beste Ausführungsform der Erfindung

Es werden Lösungen von Akrylamid, N,N'-Methylen-bis-akrylamid, Aktivatoren und Polyvinylakohol in einer physiologischen Lösung bereitgestellt, die 0,8 bis 0,9 Masse% Natriumchlorid enthält. Als Aktivatoren werden N,N'-Tetramethyläthylendiamin und Ammoniumperoxodisulfat ausgenutzt.

Aus den Lösungen wird das Reaktionsgemisch bei einem Massenverhältnis von Akrylamid, N,N'-Methylen-bis-akrylamid und Polyvinylalkohol von (15-20):(0,019-0,132):(1,0-3,0) bereitgestellt. Das Reaktionsgemisch wird in einen Reaktionsapparat eingegossen und es wird die radikalische Kopolymerisation bis zur Bildung des Polyakrylamidgels in Form von Scheiben durchgeführt. Die Gelsysteme, die bekanntlich einen hohen Feuchtigkeitsgehalt aufweisen, sind labil genug. Das im Laufe der Herstellung erreichte Gleichgewicht Polymer-flüssige Phase wird unter dem Einfluss von verschiedenen Faktoren, beispielsweise von der Temperatur und der Feuchtigkeit, gestört, Deswegen ist es notwendig, das gewonnene Polyakrylamidgel zu stabilisieren, was auf Kosten dessen zusätzlicher Strukturierung bei der Herstellung verwirklicht wird. Dazu wird in das Reaktionsgemisch Polyvinylalkohol, wünschenswert in Form von wässriger Lösung zu dessen besserer Verteilung nach der Masse, eingeführt. Die Lösung von Polyvinylalkohol in Wasser oder physiologischer Lösung stellt eine strukturierte Flüssigkeit dar.

Bei der Kopolymerisation und Gelbildung wird ein gegenseitig durchdringbares Polymernetz gebildet, das zur Stabilisierung des Gelsystems führt, dabei wird beim Gel die Hydrophilie aufrechterhalten, was sehr wichtigt ist.

Die Menge des Polyvinylalkohols wird durch die Stabilität der Grundlage in der Zeit und die Aufrechterhaltung der kennzeichnenden Kolonienbildung der beweglichen Mikroorganismenformen bedingt, die auf dem Nährmedium gewachsen sind, welches auf solch einer Grundlage bereitgestellt wurde. Die Einführung von Polyvinylalkohol in einer niedrigeren Menge, als genannt wurde, sichert die notwendige Stabilisierung der Grundlage nicht. Bei der Einführung des Polyvinylalkohols in einer höheren Menge, als angegeben wurde, verliert die Grundlage die Durchsichtigkeit, was nicht erwünscht ist. So stabilisiert Polyvinylalkohol in einer Menge im Reaktionsgemisch unter 1 g die Grundlage nicht, nach Verlauf einer Zeit wird bei der Inkubation im Thermostat bei der Temperatur 37°C die Ausschwitzung der Flüssigkeit auf der Oberfläche der Grundlage nachgewiesen, als diesem Grunde ergeben bewegliche Mikroorganismenformen kein typisches Kolonienwachstum unter Aufrechterhaltung der übrigen Eigenschaften.

Die Menge von Polyvinylalkohol über 3 g im Reaktionsgemisch stabilisiert die Grundlage derart, dass dies zur Herstellung einer sehr dichten Grundlage führt, was unerwünscht ist. Die Oberfläche des auf solch einer Grundlage bereitgestellten Platten-Nährmediums ist sehr trocken, sie wird mit der bakteriologischen Öse bei der Impfung verletzt. Die auf einem solchen Nährmedium gewachsenen Mikroorganismen weisen ein untypisches Wachstum auf, lassen sich von der Oberfläche des Nährmediums schlecht abnehmen.

Das gewonnene Gel wird als Grundlage zur Herstellung eines Platten-Nährmediums verwendet. Dazu werden die Scheiben mit der physiologischen Lösung abgewaschen und der Quellung bis zur 3,5-5fachen Vergrösserung der Masse unterzogen. Das verleiht dem Medium eine Elastizität und eine Dichte, die denen der agarisierten Medien nahekommen. Das auf diese Weise behandelte Polyakrylamidgel wird mit dem Nährsubstrat imprägiert. Als Substrat können die tryptische Nährlösung nach Hottinger, die Fleisch-Pepton-Bouillon und anderes ausgenutzt werden. Danach wird das Nahrmedium sterilisiert, getrocknet und mit Test-Mikroorganismen beimpft.

Das erhaltene Platten-Nährmedium besitzt eine stabile Struktur und gute Adhäsionseigenschaften, Elastizität und optimale Durchsichtigkeit und sichert den Effekt der Stabilität der Kultureigenschaften der Mikroorganismen von verschiedenen Arten, insbesondere der beweglichen Formen.

Nachstehend werden konkrete Beispiele der Durchführung des erfindungsgemässen Verfahrens angeführt.

Beispiel 1

Zur Gelherstellung werden vier Lösungen A, B, C, D in einer physiologischen Lösung, die 0,85 Masse% Natriumchlorid enthält, nach folgender Methodik bereitgestellt (die Komponentenmengen sind in einer Menge pro 1000 ml Lösung angegeben).

1. Herstellung der Lösung A.

Man löst 5 ml N,N'-Tetramethyläthylendiamin (TMÄD) in 995 ml physiologischer Lösung auf.

2. Herstellung der Lösung B.

0,742 g N,N'-Methylen-bis-akrylamid(MBA) werden in 500 ml physiologischer Lösung aufgelöst, auf eine Temperatur von 60°C erwärmt, es werden 470 g Akrylamid (AA) zugegeben, das bis zum vollen Auflösen vermischt wird. Die erhaltene Lösung wird abfiltriert und bis zu einem Volumen von 1000 ml mit der physiologischen Lösung gebracht.

3. Herstellung der Lösung C.

Man löst 1,78 g Ammoniumperoxodisulfat in 1000 ml physiologischer Lösung auf.

4. Herstellung der Lösung D.

Man löst 100 g Polyvinylalkohol in 900 ml physiologischer Lösung auf.

Aus den hergestellten Lösungen wird das Reaktionsgemisch bereitgestellt. Dazu werden die Lösungen A, B, C, D in folgenden Volumenverhältnissen vermischt:

A:B:C:D = 16:32:52:10

Das Reaktionsgemisch enthält Akrylamid und Polyvinylalkohol (PVA) in einem Verhältnis von 15:1. Das Reaktionsgemisch wird in den Reaktionsapparat eingegossen, wo die radikalische Kopolymerisation bis zur Bildung des Gels in Form von Scheiben vor sich geht. Nach Ablauf von 60 Minuten wird das Gel herausgenommen und mit der physiologischen Lösung innerhalb von 1 Stunde abgewaschen, danach der Quellung bis zur Massenvergrösserung um das 3,5-fache unterzogen.

Das gewonnene Gel wird als Grundlage zur Herstellung eines Platten-Nährmediums verwendet. Dazu werden die Scheiben im flüssigen Nährsubstrat-- Fleisch-Pepton-Bouillon untergebracht und für 2 bis 3 Stunden bei der Temperatur 56°C stehengelassen. Das erhaltene Nährmedium wird mit Dampf bei der Temperatur 120°C innerhalb von 30 Minuten sterilisiert und nach der Abtrocknung im Thermostat bei der Temperatur 37°C mit dem Test-Mikroorganismus beimpft.

Das Platten-Nährmedium besitzt gute Adhäsionseigenschaften, Elastizität, Dichte, Durchsichtigkeit, lässt sich mit der bakteriologischen Öse bei der Impfung der Mikroorganismen nicht verletzen.

Als flüssiges Nährsubstrat wird die Fleisch-Pepton-Bouillon verwendet, und als Test-Mikroorganismus dient Staphylococcus aureus. Nach der Inkubation der Inokulate bei der Temperatur 37°C innerhalb von 24 Stunden wachsen typische runde Kolonien von goldener Farbe mit ebenen Rändern. Das Typische der Kultur wird durch mikroskopische Untersuchungen bestätigt.

Auf dem Platten-Nährmedium bleibt das Ausschwitzen der Flüssigkeit aus. Die Kultur lässt sich vollständig mit der bakteriologischen Öse abnehmen.

Beispiel 2

Zur Herstellung des Reaktionsgemisches werden die Lösungen A, B, C, D ausgenutzt, die ähnlich wie im Beispiel 1 beschrieben bereitgestellt wurden. Die Lösungen werden in Volumenverhältnissen von A:B:C:D = 16:32:52:20 vermischt und in einen Reaktionsapparat zur Polymerisation eingegossen. Das Reaktionsgemisch enthält Akrylamid und PVA in einem Massenverhältnis von 15:2,0. Nach einer Stunde werden die Plättchen des gewonnenen Gels ähnlich wie im Beispiel 1 behandelt, indem die 5fache Vergrösserung dessen Masse erreicht wird.

Das gewonnene Gel wird mit dem Nährsubstrat ähnlich wie im Beispiel 1 imprägniert. Als Substrat wird das tryptische Medium nach Hottinger ausgenutzt, das 200 mg Stickstoff der Aminogruppe enthält; als Test-Mikroorganismus diente Bacillus pyocyaneus. Nach der Inkubation bei der Temperatur 37°C wachsen runde flache schleimartige Kolonien unter Bildung eines typischen löslichen Pigments von blau-grüner Farbe. Das Ausschwitzen der Flüssigkeit auf der Oberfläche des Platten-Nährmediums wird nicht nachgewiesen. Die Kultur wächst ins Medium nicht hinein und lässt sich mit der bakteriologischen Öse vollständig abnehem.

Beispiel 3.

Es werden die ähnlich mit dem Beispiel 1 bereitgestellten Lösungen A, C, D ausgenutzt. Zur Herstellung der Lösung B löst man 0,594 g N,N'-Methylen-bis-akrylamid in 500 ml physiologischer Lösung auf, das 0,85 Masse% Natriumchlorid enthält, gibt 470 g Akrylamid zu. Das Reaktionsgemisch wird auf ein Volumen von 1000 ml mit der physiologischen Lösung der angegebenen Konzentration gebracht.

Aus den angegebenen Lösungen wird ein Gemisch in einem Volumenverhältnis von A:B:C:D = 16:32:52:30 bereitgestellt und im Reaktionsapparat wird die Kopolymerisation durchgeführt. Das Reaktionsgemisch enthält Akrylamid und Polyvinylalkohol in einem Massenverhältnis von 15:3. Die Behandlung des gewonnenen Gels wird ähnlich mit dem Beispiel 1 durchgeführt, indem dessen 5fache Massenvergrösserung erreicht wird.

Das gewonnene Gel wird ähnlich dem Beispiel 1 mit dem Nährsubstrat imprägniert. Als Substrat wird das Medium nach Hottinger ausgenutzt, das 200 mg Stickstoff der Aminogruppe enthält. Als Test-Mikroorganismus dient Escherichia coli 055.

Nach der Indubation innerhalb von 24 Stunden bei der Temperatur 37°C wächst die Kultur in Form von glatten schwach gewöbten glänzenden Kolonien mit ebenen Rändern. Das Typische der Kultur wird bei der Untersuchung der morphologischen und agglutinablen Eigenschaften bestätigt. Das Ausschwitzen der Flüssigkeit auf der Oberfläche des Mediums bleibt aus.

Zum Vergleich werden die Kulturen E.coli 055 auf ein Platten-Nährmedium geimpft, das ähnlich wir in der Anmeldung DE, A, 3430316 bereitgestellt wurde.

Nach der Inkubation innerhalb von 24 Stunden bei der Temperatur 37°C wächst die Kultur in Form von flachen Kolonien unregelmässiger Form. Es wird das Ausschwitzen der Flüssigkeit auf einzelnen Abschnitten des Platten-Nährmediums nachgewiesen.

Beispiel 4.

Mann löst 2,4 g Polyvinylalkohol in 250 ml Wasser auf. Die gewonnene Lösung wird einer Lösung zugegossen, die 0,25 g MBA, 0,25 ml TMÄD und 159 g Akrylamid in 470 ml physiologischer Lösung enthält, die 0,9 Masse% Natriumchlorid enthält. Das erhaltene Gemisch der Lösungen wird einer Lösung zugegeben, die 0,35 g Ammoniumperoxodisulfat in 160 ml physiologischer Lösung der angegebenen Konzentration enthält, und in einen Reaktionsapparat zur Polymerisation eingegossen.

Das Reaktionsgemisch enthält AA und PVA bei einem Massenverhältnis von 20:3. Nach einer Stunde werden die Gelplatten ausgenommen, mit physiologischer Lösung abgewaschen und zur Quellung in der physiologischen Lösung bis zur 4fachen Massenvergrösserung liegengelassen.

Das gewonnene Gel wird mit einem Nährsubstrat wie im Beispiel 1 imprägniert. Als Substrat wird das Medium nach Hottinger verwendet, das 200 mg Stickstoff der Aminogruppe enthält. Als Test-Mikroorganismus dient die Kultur Salmonella typhimurium. Nach der Inkubation im Thermostat bei der Temperatur 37°C wachsen glatte schwach gewölbte Kolonien mit ebenen Rändern. Das Typische der Kultur wird bei der Untersuchung der morphologischen, zyckerlytischen und agglutinablen Eigenschaften derselben bestätigt.

Beispiel 5.

Man löst 0,8 g Polyvinylalkohol in 250 ml Wasser auf. Die gewonnene Lösung wird der Lösung zugegossen, die 0,25 g MBA, 0,25 ml TMÄD und 159 g Akrylamid in 470 ml physiologischer Lösung enthält, die 0,8 Masse% Natriumchlorid enthält. Das erhaltene Gemisch wird der Lösung zugegossen, die 0,35 g Ammoniumperoxodisulfat in 160 ml physiologischer Lösung enthält, und in einen Reaktionsapparat zur Polymerisation eingegossen.

Das Reaktionsgemisch enthält Akrylamid und Polyvinylalkohol in einem Massenverhältnis von 20:1,0. Nach einer Stunde werden die Gelplatten ausgenommen, mit physiologischer Lösung abgewaschen und der Quellung in der physiologischen Lösung bis zur 4,5fachen Massenvergrösserung unterzogen.

Das gewonnene Gel wird mit dem Nährsubstrat wie in Beispiel 1 imprägniert. Als Substrat wird das Medium nach Hottinger ausgenutzt, das 200 mg Stickstoff der Aminogruppe und 2% Glukose enthält. Als Test-Mikroorganismus dient Streptococcus pyogenes, der in Form von typischen Kolonien wächst.

Beispiel 6.

Man verwendet die Lösungen A, C, D, die ähnlich mit dem Beispiel 1 bereitgestellt wurden. Zur Herstellung der Lösung B löst man 4,125 g N,N'-Methylen-bis-akrylamid in 500 ml physiologischer Lösung auf, die 0,9 Masse% Natriumchlorid enthält, gibt man 470 g Akrylamid zu und giesst weiter physiologische Lösung der angegebenen Konzentration zu, bis ein Volumen von 1000 ml erreicht wird. Aus den genannten Lösungen wird ein Gemisch in Volumenverhältnissen von A:B:C:D = 16:32:52:15 bereitgestellt und im Reaktionsapparat wird die Kopolymerisation durchgeführt. Im Reaktionsgemisch beträgt das Massenverhältnis von Akrylamid zu N,N'-Methylen-bis-akrylamid 15:0,132 und das von Akrylamid zu Polyvinylalkohol 15:1,5.

Die Behandlung des gewonnenen Gels wird ähnlich dem Beispiel 1 durchgeführt, indem die Massenvergrösserung um das 3,5fache erreicht wird. Das erhaltene Gel wird mit dem Nährsubstrat wie im Beispiel 1 imprägniert. Als Substrat wird Fleisch-Pepton-Bouillon ausgenutzt. Als Test-Mikroorganismus dient die Kultur Shigella Flexneri. Nach der 24 Stunden langen Inkubation im Thermostat bei der Temperatur 37°C wächst die Kultur in Form von glänzenden schwach gewölbten Kolonien mit ebenen Rändern. Das Typische der Kultur wird bei der Untersuchung deren morphologischer, zuckerlytischer, agglutinabler Eigenschaften bestätigt. Die Ausschwitzung der Flüssigkeit auf der Oberfläche des Nährmediums bleibt aus.

Beispiel 7.

Man verwendet die ähnlich mit dem Beispiel 1 bereitgestellten Lösungen A, C, D. Zur Herstellung der Lösung B werden 0,594 g N,N-Methylen-bis-akrylamid in 500 ml physiologischer Lösung aufgelöst, die 0,85 Masse% Natriumchlorid enthält, 25 g Akrylamid zugegeben und weiter wird physiologische Lösung der angegebenen Konzentration zugegossen, bis ein Volumen von 1000 ml erreicht wird. Aus den genannten Lösungen wird ein Gemisch in Volumenverhältnissen von A:B:C:D = 16:32:52:30 bereitgestellt und im Reaktionsapparat wird die Kopolymerisation durchgeführt. Im Reaktionsgemisch beträgt das Massenverhältnis von Akrylamid zu N,N'-Methylen-bis-akrylamid 20:0,019 und das von Akrylamid zu Polyvinylalkohol 20:3,0.

Die Behandlung des gewonnenen Gels wird ähnlich dem Beispiel 1 vorgenommen, indem die 4,6fache Massenvergrösserung erreicht wird. Das gewonnen Gel wird mit dem Nährsubstrat wie im Beispiel 1 imprägniert. Als Nährsubstrat wurde das tryptische Medium nach Hottinger ausgenutzt, das 300 mg Stickstoff der Aminogruppe enthält. Als Test-Mikroorganismus dient die Kultur Proteus vulgaris. Nach der 24 Stunden langen Inkubation im Thermostat bei der Temperatur 37°C wächst die Kultur in Form von typischen mässig glänzenden schwach gewölbten Kolonien von der unregelmässigen Form. Das Typische der Kultur wird durch morpholobische Untersuchungen bestätigt. Es wird keine Ausschwitzung der Flüssigkeit auf der Oberfläche des Nährmediums nachgewiesen.

Beispiel 8

Man verwendet die ähnlich mit dem Beispiel 1 bereitgestellten Lösungen A, C, D. Zur Herstellung der Lösung B werden 4,135 g N,N'-Methylen-bis-akrylamid in 500 ml physiologischer Lösung aufgelöst, die 0,8 Masse% Natriumchlorid enthält, 25 g Akrylamid zugegeben und weiter wird physiologische Lösung der angebenen Konzentration bis zum Erreichen eines Volumens von 1000 ml zugegossen. Als den genannten Lösungen wird ein Gemisch in Volumenverhältnissen von A:B:C:D = 16:32:52:10 bereitgestellt und im Reaktionsapparat wird die Kopolymerisation durchgeführt. Im Reaktionsgemisch beträgt das Massenverhältnis von Akrylamid zu Polyvinylalkohol 20:1,0.

Die Behandlung des gewonnenen Gels wird ähnlich dem Beispiel 1 durchgeführt, indem die 4,0fache Massenvergrösserung erreicht wird. Das gewonnene Gel wird mit dem Nährsubstrat ähnlich dem Beispiel 1 imprägniert. Als Substrat wurde Fleisch-Pepton-Bouillon ausgenutzt. Als Test-Mikroorganismus dient Escherichia coli 0111. Nach der Inkubation der Inokulate im Thermostat bei der Temperatur 37°C wächst die Kultur in Form von glänzenden, rundlichen schwach gewölbten Kolonien mit ebenen Rändern und glatter Oberfläche. Das Typische der Kultur wird bei der Untersuchung deren morphologischer, zuckerlytischer und agglutinabler Eigenschaften bestätigt. Es wird keine Ausschwitzung der Flüssigkeit auf der Oberfläche des Nährmediums nachgewiesen.

Beispiel 9.

Man verwendet die ähnlich mit dem Beispiel 1 bereigestellten Lösungen A, C, D. Zur Hetstellung der Lösung B werden 1,78 g N,N'-Methylen-bis-akrylamid in 500 ml physiologischer Lösung aufgelöst, die 0,85 Masse% Natriumchlorid enthält, 625 g Akrylamid zugegeben und weiter physiologische Lösung der angegebenen Konzentration zugegossen, bis ein Volumen von 1000 ml erreicht wird. Aus den gennanten Lösungen wird das Gemisch in Volumenverhältnissen von A:B:C:D = 16:32:52:25 bereitgestellt. Im Reaktionsapparat führt man die Kopolymerisation durch. Im Reaktionsgemisch beträgt das Massenverhältnis von Akrylamid zu N,N'-Methylen-bis-akrylamid 20:0,057 und das von Akrylamid zu Polyvinylalkohol 20:2,5.

Die Behandlung des gewonnenen Gels wird ähnlich dem Beispiel 1 verwirklicht, indem die 4,3fache Massenvergrösserung erreicht wird. Das gewonnene Gel wird mit dem Nährsubstrat wie in Beispiel 1 imprägniert. Als Substrat wurde Nährbouillon nach Difco ausgenutzt. Als Test-Mikroorganismus diente die Kultur Bac. cereus. Nach der Inkubation innerhalb von 24 Stunden im Thermostat wächst die Kultur in Form von typischen rundlichen flachen Kolonien mit rauher Oberfläche. Das Typische der Kultur wird durch morphologische Untersuchungen bestätigt. Es wird keine Ausschwitzung der Flüssigkeit auf der Oberfläche des Nährmediums nachgewiesen.

Gewerliche Verwertbarkeit

Platten-Nährmedien, die nach dem erfindungsgemässen Verfahren hergestellt werden, können für verschiedene mikrobiologische Zwecke Verwendung finden.

## Patentansprüche

1. Verfahren zur Herstellung eines Platten-Nährmediums zur Kultivierung der Mikroorganismen, das die radikalische Kopolymerisation von Akrylamid mit N,N'-Methylen-bis-akrylamid bei deren Massenverhältnis von (15-20) : (0,019-0,132) in einer physiologischen Lösung bis zur Bildung eines Gels, das Abspülen und dessen Quellung in der physiologischen Lösung, das Imprägnieren mit einem Nährsubstrat vorsieht, dadurch **gekennzeichnet**, dass die Polymerisation in Gegenwart des Polyvinylalkohols bei einem Massenverhältnis von Akrylamid zu Polyvinylalkohol von 15-20: 1,0-3,0 durchgeführt wird.

2. Verfahren nach Anspruch 1, dadurch **gekennzeichnet**, dass die wässrige Polyvinylalkohollösung verwendet wird.

## Claims

1. A process for the preparation of a plate nutrient medium for cultivating micro-organisms, comprising the radical copolymerisation of acrylamide with N,N'-methylenebisacrylamide in a mass ratio of (15-20) : (0.019-0.132) in a physiological solution until a gel is formed, rinsing and swelling of the gel in the physiological solution, impregnation with a nutrient substrate, characterised in that polymerisation is carried out in the presence of polyvinyl alcohol in a mass ratio of acrylamide to polyvinyl alcohol of 15-20 : 1.0-3.0.

2. A process according to claim 1, characterised in that the aqueous polyvinyl alcohol solution is used.

## Revendications

1. Procédé d'obtention d'un milieu nutritif en plaque pour la culture de micro-organismes, où l'on prévoit la copolymérisation radicalaire d'acrylamide avec du N,N'-méthylène-bis-acrylamide à un rapport pondéral de (15-20) : (0,019-0,132) dans une solution physiologique jusqu'à la formation d'un gel, le lavage et son gonflement dans la solution physiologique, l'imprégnation au moyen d'un substrat nutritif, caractérisé en ce que la polymérisation est accomplie en présence d'alcool polyvinylique à un rapport pondéral de l'acrylamide à l'alcool polyvinylique de 15-20:1,0-1,3.

2. Procédé selon la revendication 1, caractérisé en ce qu'on utilise une solution aqueuse d'alcool polyvinylique.